# EUROPEAN PATENT APPLICATION

(11) **EP 4 700 793 A1**
(43) Date of publication of application: **25.02.2026**
(21) Application number: 24792440.0
(22) Date of filing: 25.03.2024
(51) Int. Cl.: G16H 50/30, G16H 50/50

(54) **DISEASE PREDICTION DEVICE, DISEASE PREDICTION METHOD, PROGRAM, AND RECORDING MEDIUM**

(30) Priority: 19.04.2023 JP 2023068450; 20.10.2023 JP 2023180785; 16.02.2024 JP 2024022230
(71) Applicant: NEC Solution Innovators, Ltd., Koto-ku, Tokyo 136-8627 (JP)
(72) Inventor: KATO Shintaro, Tokyo 136-8627 (JP); YASUDA Kosuke, Tokyo 136-8627 (JP)
(74) Representative: Betten & Resch
(86) International application number: PCT/JP2024/011785
(87) International publication number: WO 2024/219164

(57) **Abstract**

Provided is a disease prediction apparatus that enables comprehensive presentation of the health condition of the subject of disease prediction based on disease-related information. A disease prediction apparatus (10) of the present disclosure includes an information acquisition part (11), a disease prediction part (12), and an information output part (13). The information acquisition part (11) acquires disease-related information for predicting a disease for each subject of disease prediction, the disease prediction part (12) predicts a disease that the subject of disease prediction may contract in the future in each of a plurality of body parts, based on the disease-related information, to generate disease prediction information, and the information output part (13) outputs the disease prediction information.

## Description

### TECHNICAL FIELD

The present disclosure relates to a disease prediction apparatus, a disease prediction method, a program, and a recording medium.

### BACKGROUND ART

The need for preventive medicine is increasing, and individuals are required to be conscious of taking initiative in health management. Therefore, as a tool for grasping the future health condition, prediction models for myocardial infarction and the like have been disclosed (Patent Literature 1). Patent Literature 1 discloses a prediction apparatus including an acquisition part that acquires at least one feature of a patient, a prediction part that predicts a prognosis based on the at least one feature using a trained prediction model, and an output part that outputs a prediction result of the prognosis.

### Citation List

### Patent Literature

Patent Literature 1: JP 2022-184475 A

### SUMMARY

### Technical Problem

The existing prediction models like those disclosed in Patent Literature 1 focus on a single disease and do not comprehensively determine an individual's health condition.

Therefore, an example object of the present disclosure is to provide a disease prediction apparatus that enables comprehensive presentation of the health condition of the subject of disease prediction based on disease-related information.

### Solution to Problem

A disease prediction apparatus according to an example aspect of the present disclosure includes an information acquisition part that acquires disease-related information for predicting a disease for each subject of disease prediction, a disease prediction part that predicts a disease that the subject of disease prediction may contract in the future in each of a plurality of body parts, based on the disease-related information, to generate disease prediction information, and an information output part that outputs the disease prediction information.

A disease prediction method according to an example aspect of the present disclosure includes acquiring information that acquires disease-related information for predicting a disease for each subject of disease prediction, predicting disease that predicts a disease that the subject of disease prediction may contract in the future in each of a plurality of body parts, based on the disease-related information, to generate disease prediction information, and an outputting information that outputs the disease prediction information, which are executed by a computer.

A program for causing a computer to execute procedures according to an example aspect of the present disclosure includes an information acquisition procedure that acquires disease-related information for predicting a disease for each subject of disease prediction, a disease prediction procedure that predicts a disease that the subject of disease prediction may contract in the future in each of a plurality of body parts, based on the disease-related information, to generate disease prediction information, and an information output procedure that outputs the disease prediction information.

A computer-readable recording medium is recorded with a program for causing a computer to execute procedures according to an example aspect of the present disclosure. The program includes an information acquisition procedure that acquires disease-related information for predicting a disease for each subject of disease prediction, a disease prediction procedure that predicts a disease that the subject of disease prediction may contract in the future in each of a plurality of body parts, based on the disease-related information, to generate disease prediction information; and an information output procedure that outputs the disease prediction information.

### Advantageous Effects of Invention

An example advantage according to the present disclosure is that it becomes possible to comprehensively grasp the health condition of each of a plurality of body parts of the subject of disease prediction, for example, based on the disease-related information obtained through health checkup and the like.

### BRIEF DESCRIPTION OF DRAWINGS

[FIG. 1] FIG. 1 is a block diagram showing an example configuration of the disease prediction apparatus according to the present disclosure.
[FIG. 2] FIG. 2 is a block diagram showing an example hardware configuration of the disease prediction apparatus according to the present disclosure.
[FIG. 3] FIG. 3 is a flowchart showing an example processing in the disease prediction apparatus according to the present disclosure.
[FIG. 4A] FIG. 4A is a reference diagram illustrating an example of disease prediction information in the disease prediction apparatus according to the present disclosure.
[FIG. 4B] FIG. 4B is a reference diagram of examples of disease prediction information related to other diseases.
[FIG. 5A] FIG. 5A is a reference diagram illustrating an example of disease prediction change information related to lifestyle-related diseases extracted by the disease prediction apparatus according to the present disclosure.
[FIG. 5B] FIG. 5B is a reference diagram of an example of disease prediction change information related to the heart.
[FIG. 5C] FIG. 5C is a reference diagram of an example of disease prediction change information related to the brain.
[FIG. 5D] FIG. 5D is a reference diagram of an example of disease prediction change information related to the liver.
[FIG. 5E] FIG. 5E is a reference diagram of an example of disease prediction change information related to the kidney.
[FIG. 5F] FIG. 5F is a reference diagram showing an example of disease prediction information generated based on disease-related information and statistical information of risk of disease incidence after the elapse of a certain period.
[FIG. 6] FIG. 6 is a block diagram showing an example configuration of the disease prediction apparatus according to the present disclosure.
[FIG. 7] FIG. 7 is a flowchart showing an example processing in the disease prediction apparatus according to the present disclosure.
[FIG. 8A] FIG. 8A is a reference diagram illustrating an example of the risk of disease onset for generating recommended examination information in the disease prediction apparatus according to the present disclosure.
[FIG. 8B] FIG. 8B is a reference diagram illustrating an example of the recommended examination information in the disease prediction apparatus according to the present disclosure.
[FIG. 9] FIG. 9 is a block diagram showing an example configuration of the disease prediction apparatus according to the present disclosure.
[FIG. 10] FIG. 10 is a flowchart showing an example processing in the disease prediction apparatus according to the present disclosure.
[FIG. 11A] FIG. 11A is a reference diagram illustrating an example of an analysis method used for analysis in the disease prediction apparatus according to the present disclosure.
[FIG. 11B] FIG. 11B is a reference diagram according to another example of the analysis method.
[FIG. 12] FIG. 12 is a reference diagram illustrating an example of high-risk attribute information extracted by the disease prediction apparatus according to the present disclosure.

### EXAMPLE EMBODIMENTS

The example embodiments of present disclosure is described below with reference to the drawings. It is to be noted, however, that the present disclosure is by no means limited or restricted by the following example embodiments. In the following drawings, identical parts are indicated with identical reference signs. Regarding the descriptions of the example embodiments, reference can be made to one another unless otherwise stated. Furthermore, the configurations of the example embodiments can be combined unless otherwise stated.

### First Example Embodiment

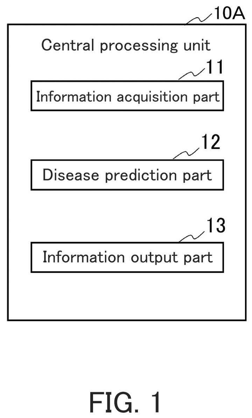

FIG. 1 is a block diagram showing an example configuration of the disease prediction apparatus 10A according to the present disclosure (hereinafter also referred to as "the apparatus 10A"). As shown in FIG. 1, the apparatus 10A includes an information acquisition part 11, a disease prediction part 12, and an information output part 13. Although not shown, the apparatus 10A may include, for example, an input part, an output part, a display, and/or a storage.

The apparatus 10A may be, for example, one apparatus including the aforementioned parts, or may be an apparatus to which the respective parts are connectable via a communication line network. Further, the apparatus 10A is connectable to an external device described later, via the communication line network. The communication line network is not particularly limited and may be a known network and may be, for example, wired or wireless. Examples of the communication line network include an internet line, WWW (World Wide Web), a telephone line, LAN (Local Area Network), SAN (Storage Area Network), DTN (Delay Tolerant Networking), LPWA (Low Power Wide Area), and L5G (local 5G). Examples of wireless communication include WI-FI^{®} (registered trademark), BLUETOOTH^{®} (registered trademark), local 5G, and LPWA. The wireless communication may be a form where apparatuses directly communicate with each other (Ad-Hoc communication), infrastructure communication, or may be indirect communication via an access point or the like. The apparatus 10A may be built in a server of a system, for example. The apparatus 10A may also be, for example, a personal computer (PC, e.g., a desktop PC and a laptop), a smartphone, a tablet terminal, or the like with the program of the present disclosure installed therein. The apparatus 10A may be in a form of, for example, cloud computing, edge computing, or the like such that at least one of the aforementioned parts is on a server and the rest of the parts are on a terminal.

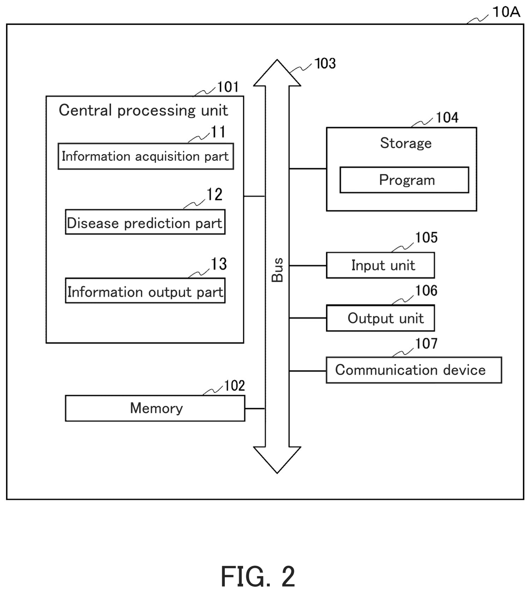

FIG. 2 is a block diagram showing an example hardware configuration of the apparatus 10A. The apparatus 10A includes, for example, a central processing unit (CPU, GPU, etc.) 101, a memory 102, a bus 103, a storage 104, an input unit 105, an output unit 106, a communication device 107, and the like. The units of the apparatus 10A are connected to each other by their respective interfaces (I/F), via the bus 103.

The central processing unit 101 operates in cooperation with other configurations with a controller (a system controller, an I/O controller, or the like), and is responsible for control of the entire apparatus 10A. In the apparatus 10A, the central processing unit 101 executes, for example, the program of the present disclosure or other programs, and reads and writes various data. Specifically, for example, the central processing unit 101 functions as the information acquisition part 11, the disease prediction part 12, and the information output part 13. When the apparatus 10A includes the output part, the central processing unit 101 may function as the output part. The apparatus 10A may include another arithmetic unit such as a CPU, a GPU (Graphics Processing Unit) and an APU (Accelerated Processing Unit), or a combination of these and the CPU.

The bus 103 is connectable to an external device, for example. Examples of the external device include a terminal of a user, an external storage (external data base, etc.), a printer, an external input unit, an external display, and an external imaging device. The apparatus 10A can be, for example, connected to an external network (the communication line network) through the communication device 107 connected to the bus 103, and can be connected to other devices via the external network.

The memory 102 may be, for example, a main memory (main storage). When the central processing unit 101 executes processing, for example, the memory 102 reads various operation programs such as the program of the present disclosure stored in the storage 104 to be described later, and the central processing unit 101 receives the data from the memory 102 to execute the program. The main memory is, for example, a RAM (Random-Access Memory). The memory 102 may be, for example, a ROM (Read-Only Memory).

The storage 104 is also referred to as a so-called auxiliary storage with respect to the main memory (main storage), for example. As mentioned above, the storage 104 stores operation programs including the program of the present disclosure. The storage 104 may be, for example, a combination of a recording medium and a drive for performing reading and writing on a recording medium. The recording medium is not particularly limited and may be a built-in type or an external type, and examples thereof include HD (Hard Disk), CD-ROM, CD-R, CD-RW, MO, DVD, a flash memory, and a memory card. The storage 104 may be, for example, a hard disk drive (HDD) or a solid state drive (SSD), in which a recording medium and a drive are integrated. When the apparatus 10A includes the storage, for example, the storage 104 functions as the storage. The storage can store, for example, disease-related information, disease prediction information, recommended examination information, and disease prediction change information, etc., which will be described later.

In the apparatus 10A, the memory 102 and the storage 104 can also store various information such as log information, information acquired from an external data base (not shown) or an external device, information generated by the apparatus 10A, and information used when the apparatus 10A executes processing. In this case, the memory 102 and the storage 104 may store, for example, the disease-related information. It should be noted that, at least a piece of the information may be, for example, stored in an external server other than the memory 102 and the storage 104, or may be dispersedly stored in a plurality of terminals using a blockchain technique or the like.

The apparatus 10A further includes, for example, an input unit 105 and an output unit 106. Examples of the input unit 105 include a pointing device such as a touch panel, a track pad, and a mouse; a keyboard; an imaging device such as a camera and a scanner; a card reader such as an IC card reader and a magnetic card reader; and an audio inputting device such as a microphone. Examples of the output unit 106 include a display such as an LED display and a liquid crystal display; an audio output device such as a speaker; and a printer. In the present disclosure, the input unit 105 and the output unit 106 are configured separately, but the input unit 105 and the output unit 106 may be configured integrally like a touch panel display.

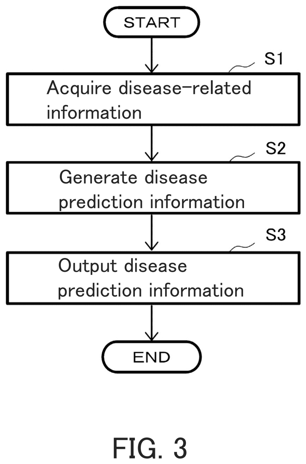

Next, an example of the disease prediction method according to the present disclosure will be described with reference to the flowchart of FIG.3. The disease prediction method according to the present disclosure may be performed, for example, as follows using the apparatus 10A shown in FIG. 1 or 2. Note that, the disease prediction method according to the present disclosure is not limited to the use of the apparatus 10A in FIG. 1 or 2.

First, the information acquisition part 11 acquires disease-related information for predicting a disease for each subject of disease prediction (S1, information acquisition). The disease-related information is, for example, medical record information, health checkup information, and attribute information of the subject of disease prediction (sex, age, etc.). The disease-related information is not limited to the examples mentioned above, and may be in any form as long as information necessary for disease prediction can be obtained. Examples of the method for acquiring the disease-related information include, but are not limited to, a method in which the disease-related information is acquired from a connected external database, a method in which the disease-related information is acquired via a communication line, and a method in which necessary information items are directly input to the apparatus 10A.

Next, the disease prediction part 12 predicts a disease that the subject of disease prediction may contract in the future in each of a plurality of body parts, based on the disease-related information, to generate disease prediction information (S2, disease prediction). The disease prediction information indicates, for example, the name of a disease with a likelihood of incidence, but is not limited thereto, and may include reference information such as information on comparison with a standard risk for subjects of the same sex and age. In addition, the disease prediction information may include risk score information indicating the likelihood of disease incidence. The risk score is a measure of the likelihood of disease incidence. The risk score is used for the determination of necessity for detailed examination, comparison with the standard risk, and the like. The risk score indicates the likelihood of disease incidence by a numerical value, and the units of the numerical value and the like may be appropriately set according to the intended use. The prediction of the likelihood of disease incidence is performed, for example, based on the association between each factor used for predicting a disease and the disease itself, factors with a high likelihood of disease incidence, and the correlation between a disease to be predicted and other diseases, using analysis results of the likelihood of disease incidence based on the state of each factor included in the disease-related information. Examples of the factor used for predicting a disease incidence include age, sex, height, weight, good cholesterol (HDL), bad cholesterol (LDL), triglycerides (TG), γ-GTP, AST(GOT), ALT(GPT), BMI, systolic blood pressure, diastolic blood pressure, blood glucose level, lifestyle habits, medical history, and medication information. Note that the factors are not limited to those described above, and other information may be added. Not all the factors according to the above examples are necessarily required, and they may be appropriately set according to the intended purpose and the like. Examples of the disease to be predicted include diseases related to the brain, heart, liver, kidney, endocrine system, metabolism, nerves, blood vessels, bones, joints, and muscles, but are not limited thereto, and may be set as appropriate. The disease prediction is generated for each of a plurality of body parts. For example, as the brain-related diseases, prediction of incidence of cerebral infarction, cerebral hemorrhage, subarachnoid hemorrhage, and the like is performed; as the heart-related diseases, prediction of incidence of myocardial infarction, angina pectoris, heart failure, chronic ischemic heart disease, atrial fibrillation, atrial flutter, and the like is performed; as the liver-related diseases, prediction of incidence of fatty liver, cirrhosis, alcoholic liver disease, liver fibrosis, and the like is performed; as the kidney-related diseases, prediction of incidence of diabetic nephropathy, chronic kidney disease (CKD), and the like is performed; as the endocrine system-related diseases, prediction of incidence of type 2 diabetes and the like is performed; as the metabolism-related diseases, prediction of incidence of lipoprotein metabolism disorders, other lipidemias, and the like, purine and pyrimidine metabolism disorders (hyperuricemia) is performed; as the nerves-related diseases, prediction of incidence of sleep disorders and the like is performed; as the blood vessel-related diseases, prediction of incidence of essential hypertension, atherosclerosis, aortic aneurysm, aortic dissection, and the like is performed; as the bone-related diseases, prediction of incidence of osteoporosis and the like is performed; as the joint-related diseases, prediction of incidence of gout, knee arthritis, and the like is performed; and as the muscle-related diseases and the like, prediction is also performed. The disease to be predicted is not limited to the examples described above, and may be set as appropriate.

In the case where past disease prediction information exists, comparison with the past disease prediction information may be performed. The disease prediction part 12 may refer to past disease prediction information, extract a content that has changed in comparison with the past disease prediction information from current disease prediction information, and generate the content as disease prediction change information. The past disease prediction information may be a single piece of information or a plurality of pieces of information. The disease prediction change information may be obtained, for example, by extracting only the content that has changed in comparison with the past disease prediction information, or may also include the content that has not changed. In the case where a plurality of pieces of past disease prediction information exist, the disease prediction change information may, for example, indicate a change history of the disease prediction information.

The disease prediction part 12 may predict, for example, a disease that the subject of disease prediction may contract in the future in each of a plurality of body parts, based on the disease-related information and statistical information on risk of disease incidence after a certain period has elapsed, to generate the disease prediction information. The risk of disease incidence after a certain period has elapsed is, for example, the risk of disease incidence associated with aging. Also, the certain period is not particularly limited, and may be, for example, a period of a certain number of years. The certain number of years may be, for example, 1 year, 5 years, 10 years, 20 years, 30 years, 40 years, or 50 years, but is not limited to these. The disease prediction information may be generated using, for example, a trained model that includes the statistical information. By generating disease prediction information through predicting a disease that the subject of disease prediction may contract in the future in each of a plurality of body parts, based on the disease-related information and statistical information on risk of disease incidence after a certain period has elapsed, it is possible to predict diseases that the subject of disease prediction may contract in the future if the current test values and lifestyle habits remain unchanged over time. Therefore, it is particularly effective for young people in their 20s to 30s to prevent future diseases.

Next, the information output part 13 outputs the disease prediction information (S3, information output). When the disease prediction part 12 generates disease prediction change information, the information output part 13 may output the disease prediction change information. In this case, the information output by the information output part 13 may be only the disease prediction information, only the disease prediction change information, or both of them. The disease prediction information is output, for example, by being displayed on a dedicated monitor connected as an external device. The output is not limited thereto, and for example, may be transmission to a terminal of the subject of disease prediction via a communication line, or output to an external database, and may be appropriately set according to the intended purpose.

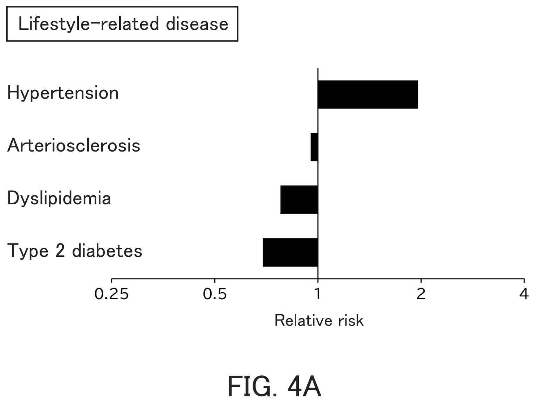

Hereinafter, an example of a specific flow of disease prediction using the apparatus 10A will be described with reference to FIGs. 4A to 5E. Note that the following description is an example of a specific flow, and as described above, the apparatus 10A and the disease prediction method of the present disclosure are not limited to the following description, and the disease prediction method of the present disclosure is not limited to the use of the apparatus 10A. The diseases described below are merely examples, and the present disclosure is not limited to these.

First, disease-related information is input based on the health checkup results of the subject of disease prediction. The provided disease-related information is acquired by the information acquisition part 11 of the apparatus 10A. Next, the disease prediction part 12 generates disease prediction information based on the disease-related information acquired by the information acquisition part 11, using the analysis results of the likelihood of disease incidence provided by the apparatus 10A.

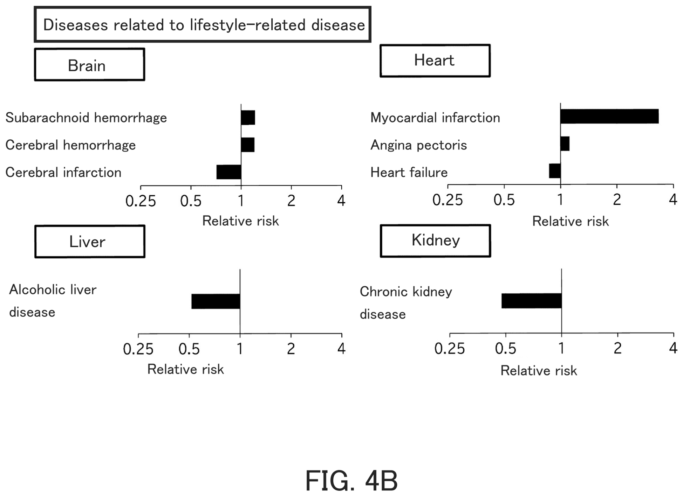

Examples of the disease prediction information are shown in FIGs. 4A and 4B. FIG. 4A shows disease prediction information on the risk of lifestyle-related disease. The vertical axis of the table indicates the name of lifestyle-related disease, and the horizontal axis indicates the relative risk compared to subjects of the same sex and age group. The vertical line at the point marked "1" on the horizontal axis indicates the standard value, with the bar graph extending to the right indicating a higher relative risk, and the bar graph extending to the left indicating a lower relative risk. The standard value may be, for example, a median value or an average value of subjects of the same sex and age group, and may be appropriately set according to actual circumstances. Furthermore, the display of the disease prediction information is not limited to the display based on the relative risk, but may be, for example, a display based on the risk score or a display of the risk of disease onset. The risk of disease onset may be, for example, a risk of onset within 4 years, but may be appropriately changed to, for example, a risk of onset within 10 years or another period as appropriate. FIG. 4A shows only the prediction of incidence of diseases related to lifestyle-related disease, and no information is shown for diseases affecting other parts of the body. The apparatus 10A can also perform prediction of incidence of diseases related to the brain, heart, liver, and kidneys based on the same disease-related information as shown in FIG. 4B. The explanation of the configuration of each table in FIG. 4B is the same as that given for FIG. 4A. As a result, the subject of disease prediction can grasp, based on FIGs. 4A and 4B, that, in addition to hypertension, the likelihood of myocardial infarction incidence is higher than the standard risk level.

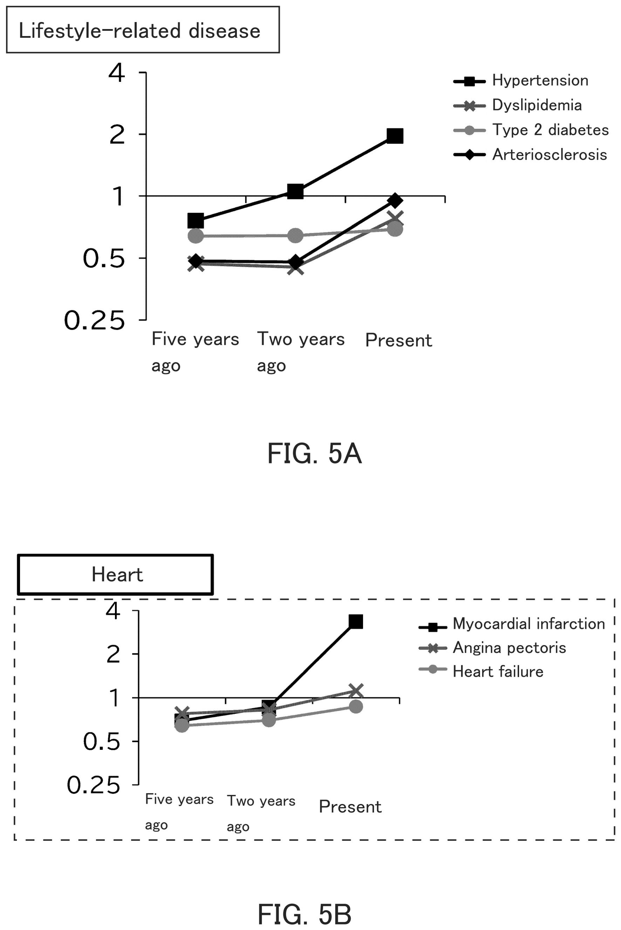

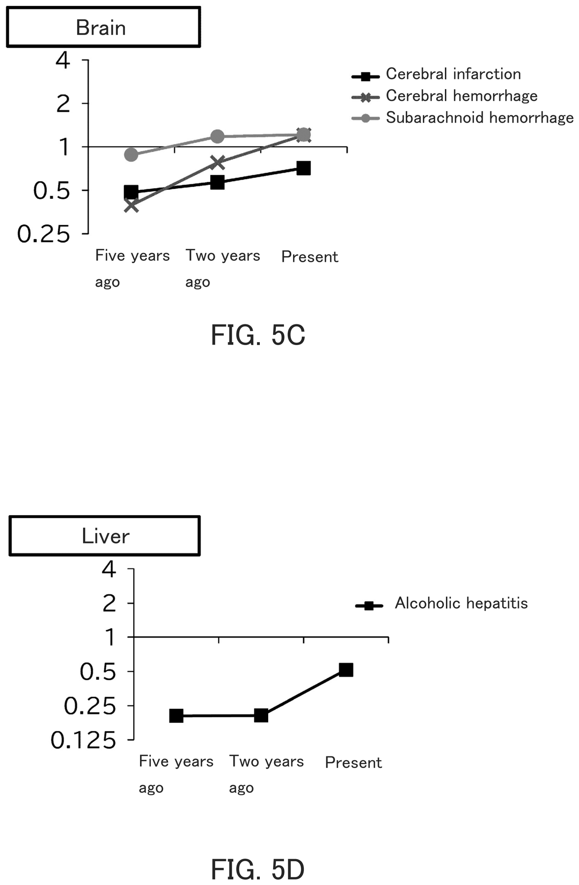

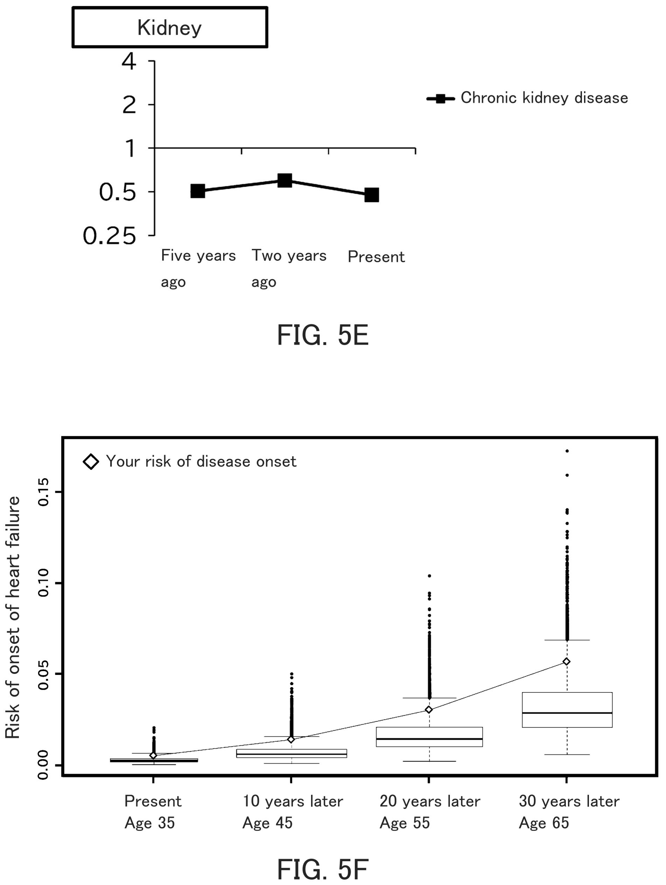

In addition, by referring to past disease prediction information, changes in the current disease prediction information can be grasped. In each of FIGs. 5A to 5E, the vertical axis indicates the relative risk, and the transition of relative risk for each disease is shown as a line graph. The relative risk indicates a ratio to the standard risk where 1.0 indicates the standard risk for subjects of the same sex and age group, and for example, 1.2 indicates that the risk of the subject of disease prediction is 1.2 times the standard risk. In each of FIGs. 5A to 5E, the numerical values corresponding to 5 years ago and 2 years ago are based on the past disease prediction information, and the numerical values corresponding to the present are based on the current disease prediction information, indicating changes from the past disease prediction information. FIG. 5A shows that the risk of hypertension continues to increase, as it did previously, and should be carefully monitored. In addition, FIG. 5B, enclosed by a dashed line, shows that, unlike the previous trend, the risk of myocardial infarction has increased sharply in terms of relative risk and now significantly exceeds the standard risk level. This indicates that, in addition to the prior concerns, new concerns have arisen. This reveals that the subject of disease prediction now needs to pay attention to heart-related diseases as well. Note that if a plurality of pieces of past disease prediction information are available, it is possible to grasp the progression in more detail.

Furthermore, another example of the disease prediction information is shown in FIG. 5F. FIG. 5F is a reference diagram illustrating an example of disease prediction information generated based on disease-related information and statistical information on the risk of disease incidence after a certain period of time has elapsed. In FIG. 5F, the vertical axis indicates the risk of disease onset, and the horizontal axis indicates the number of years. Heart failure is shown as an example of the disease. As shown in FIG. 5F, the risk of onset of heart failure, based on health checkup results at present (age 35) and lifestyle habit information, is not significantly different from that of subjects of the same age group. On the other hand, if the current health checkup results and lifestyle habits remain unchanged at 10 years later (age 45 ), 20 years later (age 55), and 30 years later (age 65), the risk of onset of heart failure is expected to exceed that of the subjects of the same age group. This enables, in particular, younger generations to understand their future risks of disease onset at an early stage and take preventive action.

The disease prediction apparatus of the present disclosure can grasp the health condition of each of a plurality of body parts of the subject of disease prediction based on disease-related information obtained through health checkups and the like. This enables subject of disease prediction to comprehensively manage their own health condition.

### Second Example Embodiment

The disease prediction apparatus of the present disclosure generates recommended examination information. Hereinafter, a description will be given of a disease prediction apparatus with a configuration for recommended examination information generation added to the first example embodiment, which may also perform statistical analysis as described later.

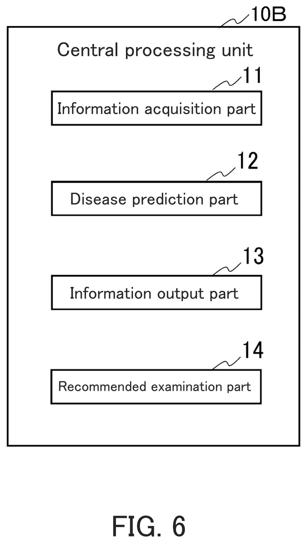

The disease prediction apparatus of the present disclosure will be described with reference to FIG. 6. FIG. 6 is a block diagram showing an example configuration of the disease prediction apparatus 10B according to the present disclosure (hereinafter also referred to as the "apparatus 10B"). As shown in FIG. 6, the apparatus 10B of the present disclosure includes, for example, a recommended examination information generation part 14 in addition to the configuration (information acquisition part 11, disease prediction part 12, and information output part 13) of the apparatus 10A of the first example embodiment. In the apparatus 10B, for example, the configuration other than the inclusion of the recommended examination information generation part 14, including the hardware configuration, is the same as that of the apparatus 10A of the first example embodiment, and thus reference can be made to the description of the first example embodiment.

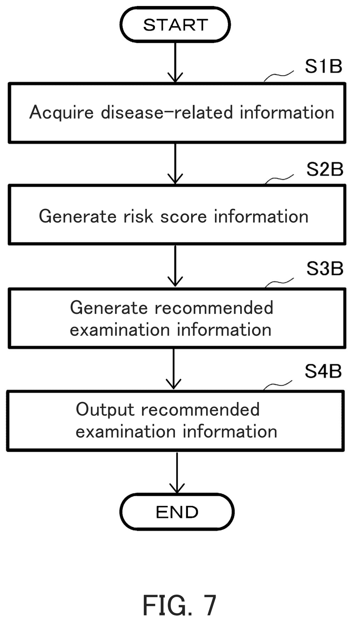

Next, an example of the disease prediction method according to the present disclosure will be described with reference to the flowchart of FIG. 7. The disease prediction method according to the present disclosure is performed, for example, as follows using the apparatus 10B shown in FIG. 6. Note that, the disease prediction method according to the present disclosure is not limited to the use of the apparatus 10B in FIG. 6.

The information acquisition part 11 of the apparatus 10B of the present disclosure acquires disease-related information for predicting a disease for each subject of disease prediction (S1B, information acquisition). Next, the disease prediction part 12 predicts a disease that the subject of disease prediction may contract in the future in each of a plurality of body parts, based on the disease-related information, to generate disease prediction information including risk score information (S2B, disease prediction). The information acquisition and the disease prediction are the same as those in the first example embodiment, and thus reference can be made to the description of the first example embodiment.

Next, the recommended examination information generation part 14 generates recommended examination information related to an examination recommended for the subject of disease prediction based on the risk score for each of the plurality of body parts (S3B, recommended examination information). The recommended examination information refers to information that encourages a detailed examination by displaying, based on the risk score, for example, the names of examinations necessary for a disease for which the subject of disease prediction has a higher risk score than the standard risk score of subjects of the same sex and age group. The items displayed as the recommended examination information are not limited to the names of examinations, and may be set as appropriate by adding items useful for the subject of disease prediction, such as the name of the disease recommended for detailed examination, preventive actions to be taken, and precautions, and the like. Furthermore, the criteria for recommending examinations and the like in the recommended examination information are not limited to cases where the subject of disease prediction has a risk score higher than the standard risk score, and may be set as appropriate. For example, depending on the type of disease, even when the risk score of the subject of disease prediction is lower than the standard risk score, if the risk score is higher than a certain risk score threshold, a detailed examination may be recommended.

Next, the information output part 13 outputs recommended examination information (S4B, information output). The output method by the information output part 13 is the same as that in the first example embodiment, and thus reference can be made to the description of the first example embodiment. Furthermore, the information acquisition part 13 may output the disease prediction information and the disease prediction change information together.

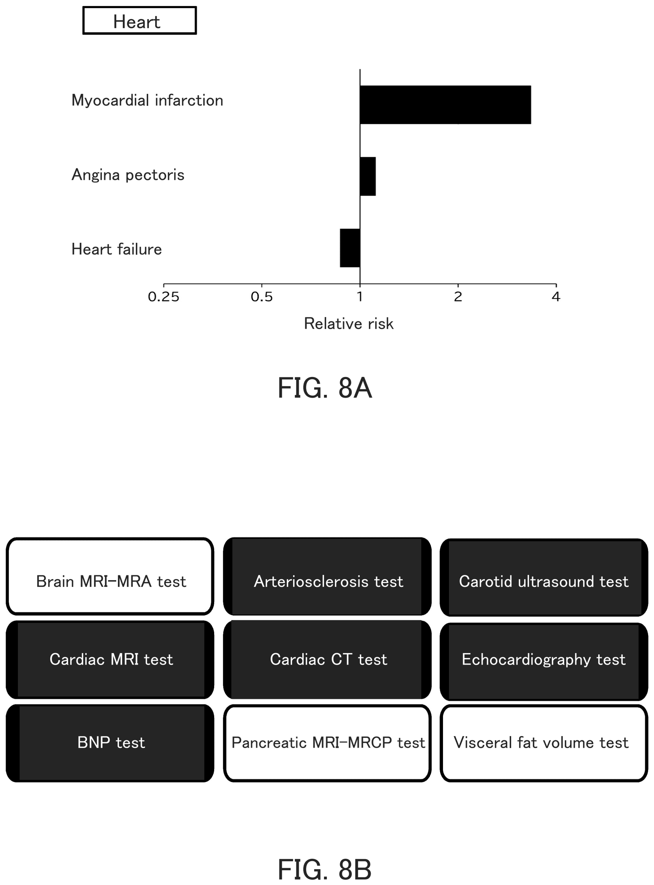

Hereinafter, an example of a specific flow of disease prediction using the apparatus 10B will be described with reference to FIGs. 8A and 8B. Note that the following description is an example of a specific flow, and as described above, the apparatus 10B and the disease prediction method of the present disclosure are not limited to the following description, and the disease prediction method of the present disclosure is not limited to the use of the apparatus 10B. The diseases described below are merely examples, and the present disclosure is not limited to these.

First, the flow up to the generation of disease prediction information is the same as that in the first example embodiment, and thus reference can be made to the description of the first example embodiment. FIG. 8A is a diagram concerning the heart shown in FIG. 4B. FIG. 8B shows an example of displaying recommended examinations based on the result of FIG. 8A. In the display of FIG. 8B, examination names written in black characters on white backgrounds indicate examinations that are not recommended, and examination names written in white characters on black backgrounds indicate recommended examinations. In FIG. 8A, the bar graph corresponding to myocardial infarction extends to the right, which indicates that the relative risk is higher than the standard risk. Therefore, the display of FIG. 8B indicates that arteriosclerosis test, carotid ultrasound test, cardiac MRI test, cardiac CI test, echocardiography test, and BNP test are recommended as examinations corresponding to myocardial infarction where the relative risk exceeds a certain threshold. In this way, the apparatus 10B can show information about recommended examinations based on the risk score generated by the disease prediction part 12, for example, when the relative risk exceeds a certain threshold.

The apparatus 10B of the present disclosure shows recommended examinations in detail based on disease prediction for each of a plurality of body parts of the subject of disease prediction, thus it is possible to encourage the subject of disease prediction to undergo appropriate examinations. The apparatus 10B of the present disclosure can also be used to determine which medical department the subject should visit based on the results of general health checkup.

### Third Example Embodiment

The disease prediction apparatus of the present disclosure performs analysis based on statistical information. Hereinafter, a description will be given of a disease prediction apparatus with a configuration for statistical analysis added to the first example embodiment, which may also perform recommended examination information generation as in the second example embodiment.

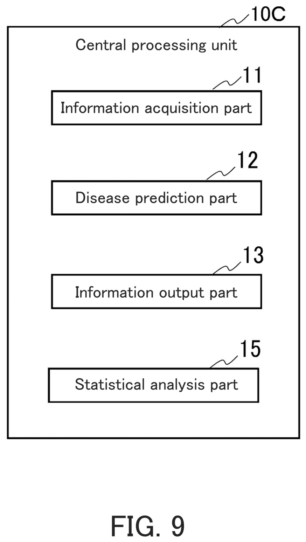

The disease prediction apparatus of the present disclosure will be described with reference to FIG. 9. FIG. 9 is a block diagram showing an example configuration of the disease prediction apparatus 10C according to the present disclosure (hereinafter also referred to as the "apparatus 10C"). As shown in FIG. 9, the apparatus 10C of the present disclosure includes, for example, a statistical analysis part 15 in addition to the configuration (information acquisition part 11, disease prediction part 12, and information output part 13) of the apparatus 10A of the first example embodiment. In the apparatus 10C, for example, the configuration other than the inclusion of the statistical analysis part 15, including the hardware configuration, is the same as that of the apparatus 10A of the first example embodiment, and thus reference can be made to the description of the first example embodiment.

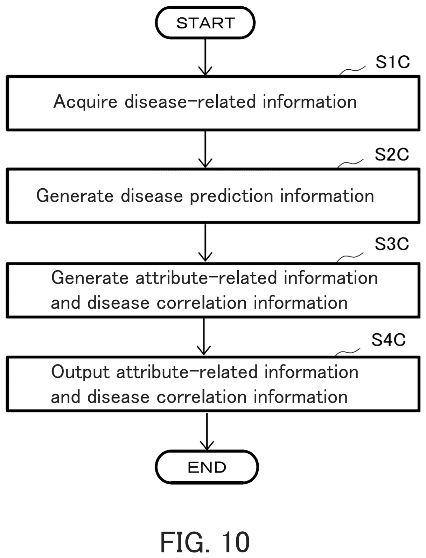

Next, an example of the disease prediction method according to the present disclosure will be described with reference to the flowchart of FIG. 10. The disease prediction method according to the present disclosure is performed, for example, as follows using the apparatus 10C shown in FIG. 9. Note that the disease prediction method according to the present disclosure is not limited to the use of the apparatus 10C in FIG. 9.

The information acquisition part 11 of the apparatus 10C of the present disclosure acquires disease-related information for predicting a disease for each subject of disease prediction (S1C, information acquisition). Next, the disease prediction part 12 predicts a disease that the subject of disease prediction may contract in the future in each of a plurality of body parts, and generates disease prediction information based on the disease-related information (S2C, disease prediction). The information acquisition and the disease prediction are the same as those in the first example embodiment, and thus reference can be made to the description of the first example embodiment.

Next, the statistical analysis part 15 statistically analyzes the disease prediction information of a plurality of subjects of disease prediction, analyzes an association between a type of disease and an attribute of the subject of disease prediction, and generates attribute-related information (S3C-1, statistical analysis). The types of disease correspond to the diseases to be predicted in the present disclosure including the first and second example embodiments. Furthermore, the attributes of the subjects of disease prediction correspond to factors for predicting the likelihood of disease incidence. The disease prediction information on a plurality of subjects of disease prediction is accumulated, and through statistical analysis, for example, the association between diseases and age is analyzed. Information about the above association for the plurality of subjects of disease prediction is then generated as attribute-related information.

Furthermore, the statistical analysis part 15 may further extract an attribute with a high likelihood of disease incidence, based on the attribute-related information, to generate high-risk attribute information (S3C-2, statistical analysis).

In addition, the statistical analysis part 15 may statistically analyze the disease prediction information of a plurality of subjects of disease prediction, analyze a correlation between diseases, and generate disease correlation information (S3C-3, statistical analysis).

Next, the information output part 13 outputs the attribute-related information, the high-risk attribute information, and the disease correlation information in response to the information generated by the statistical analysis part 15 (S4C, information output). The output method by the information output part 13 is the same as that in the first example embodiment, and thus reference can be made to the description of the first example embodiment. Also, the information output part 13 may output disease prediction information as well.

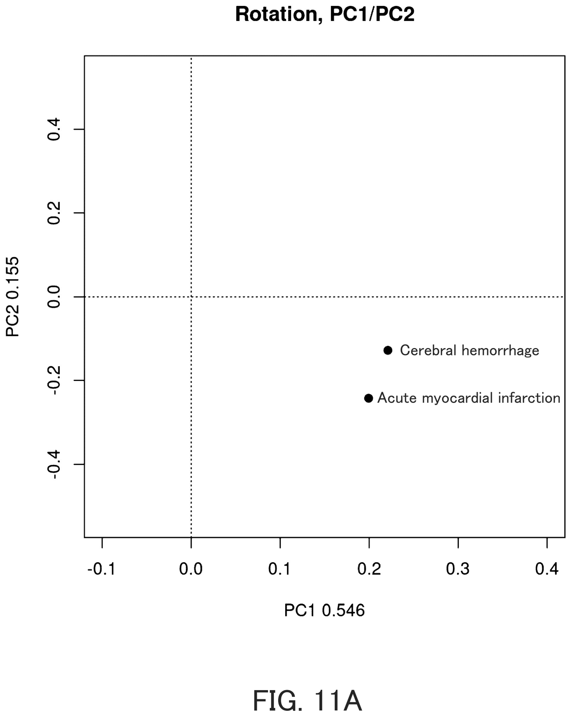

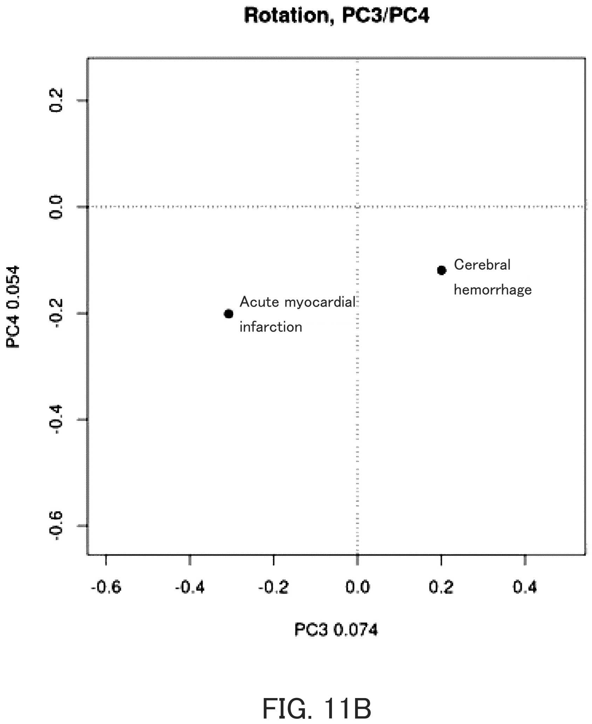

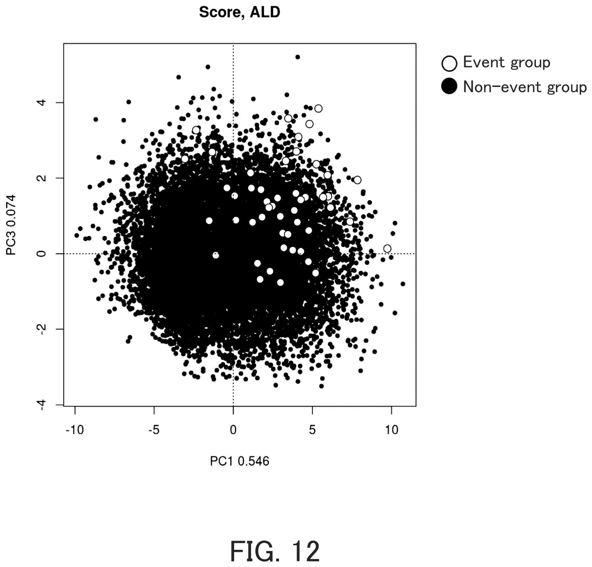

Hereinafter, an example of a specific flow of disease prediction using the apparatus 10C will be described with reference to FIGs. 11 and 12. Note that the following description is an example of a specific flow, and as described above, the apparatus 10C and the disease prediction method of the present disclosure are not limited to the following description, and the disease prediction method of the present disclosure is not limited to the use of the apparatus 10C. The diseases described below are merely examples, and the present disclosure is not limited to these.

First, the flow up to the generation of disease prediction information is the same as that in the first example embodiment, so that reference can be made to the description of the first example embodiment. The disease prediction information generated as a result is stored in, for example, an external database connected to the apparatus 10C. Statistical analysis is performed based on the aforementioned accumulated disease prediction information.

The model for statistical analysis is generated, for example, as follows. Diseases to be predicted are selected, and the data is organized. The diseases to be predicted are selected, for example, based on ICD codes, and mainly chosen from diseases for which therapeutic or preventive effects are expected, such as cardiovascular disease, endocrine disease, and kidney disease. The data is processed by removing outliers, and, for example, health checkup information and diagnostic information are linked using personal IDs, and the time to disease onset is calculated based on health checkup information. The subjects are, for example, those who have no diagnostic records at the time of their first health checkup, and those with a history of medication use for diabetes, hypertension, or dyslipidemia based on questionnaire responses are excluded. From the health checkup information and diagnostic information, for example, 50 percent is randomly used as training data, 25 percent is used as evaluation data, and the remaining 25 percent is used for final evaluation. A prediction model is generated by using training data, for example, by applying a Cox proportional hazards model, and selecting factors through stepwise selection and the Akaike Information Criterion (AIC). After the generation of the prediction model, for example, evaluation is performed using AUC based on evaluation data, and after the prediction model is finalized, a final evaluation is conducted.

When the association between the type of disease and the attribute of subject of disease prediction is analyzed using the prediction model generated as described above, for example, principal component analysis as in FIGs. 11A and 11B can be performed. In FIG. 11A, the vertical axis indicates the first principal component, which is highly associated with sex, and the horizontal axis indicates the second principal component, which is highly associated with age. FIG. 11B shows the distribution based on the third and fourth principal components, which are different from the principal components associated with age and sex. Note that, although only myocardial infarction and cerebral hemorrhage are shown in FIGs. 11A and FIG. 11B for clarity, other diseases can also be displayed in practice. According to FIGs. 11A and 11B, myocardial infarction and cerebral hemorrhage appear to be highly correlated in terms of association with age and sex but weakly correlated when the influence of age and sex is partially removed. In this way, statistical analysis enables the analysis of the association between the type of disease and the attribute of subject of disease prediction.

Furthermore, statistical analysis enables the extraction of attributes with a high likelihood of disease incidence, thereby allowing the generation of high-risk attribute information. FIG. 12 is a distribution map showing the distribution of subjects at high risk of disease incidence, where white circles indicate those who have developed the disease. The horizontal axis is set to the axis highly associated with age, as in FIG. 11A. The vertical axis is set to an axis that is less influenced by age and sex. In FIG. 12, those who have developed the disease are particularly concentrated in the upper-right region, which indicates that this region is a high-risk area.

Then, although it is not shown, based on the results of statistical analysis, a distribution map is created for two diseases, showing subjects with high risk of one disease and subjects with high risk of both diseases. This makes it possible to confirm the correlation between the two diseases. In this way, statistical analysis enables the analysis of correlation between diseases.

In addition to the disease prediction information, the apparatus 10C of the present disclosure can show objective state based on statistics. This makes it possible to assess the health condition of the subject of disease prediction in a more objective manner.

### Fourth Example Embodiment

The program according to the present disclosure is a program for causing a computer to execute each of the processes of the present disclosure described above. Specifically, the program according to the present disclosure is a program for causing a computer to execute, for example, an information acquisition procedure, a disease prediction procedure, and an information output procedure.

The information acquisition procedure acquires disease-related information for predicting a disease for each subject of disease prediction, the disease prediction procedure predicts a disease that the subject of disease prediction may contract in the future in each of a plurality of body parts, based on the disease-related information, to generate disease prediction information, and the information output procedure outputs the disease prediction information.

The program according to the present disclosure can also be regarded as a program that causes a computer to function as, for example, the information acquisition procedure, the disease prediction procedure, and the information output procedure.

The program according to the present disclosure can incorporate the descriptions given in the disease prediction apparatus and the disease prediction method according to the first to third example embodiments of the present disclosure. In each of the processes, for example, "procedure" and "processing" can be interchangeably used. The program according to the present disclosure may be recorded in a computer-readable recording medium, for example. The recording medium is, for example, a non-transitory computer-readable recording medium (a non-transitory computer-readable storage medium). The recording medium is not particularly limited and examples thereof include a random access memory (RAM), a read only memory (ROM), a hard disk (HD), a flash memory (e.g., SSD (Solid State Drive), a USB flash memory, and a SD/SDHC card), an optical disc (e.g., CD-R/CD-RW, DVD-R/DVD-RW, and BD-R/BD-RE), a magneto-optical disk (MO), and a FLOPPY^{®} (registered trademark) disk (FD). The program (may be referred to as a programming product or a program product, for example) according to the present disclosure may be in a form to be distributed from an external computer, for example. The "distribution" may be distribution via a communication line network or distribution via a wired-connected device. The program according to the present disclosure may be executed by being installed in a device where the program is distributed, or may be executed without being installed.

While the present disclosure has been particularly shown and described with reference to example embodiments thereof, the present disclosure is not limited to these embodiments. It will be understood by those of ordinary skill in the art that various changes in form and details may be made therein without departing from the spirit and scope of the present disclosure as defined by the claims.

### SUPPLEMENTARY NOTE

The whole or part of the example embodiments disclosed above can be described as, but not limited to, the following supplementary notes.

### (Supplementary Note 1)

A disease prediction apparatus, including:
an information acquisition part;
a disease prediction part; and
an information output part, wherein
the information acquisition part acquires disease-related information for predicting a disease for each subject of disease prediction,
the disease prediction part predicts a disease that the subject of disease prediction may contract in the future in each of a plurality of body parts, based on the disease-related information, to generate disease prediction information, and
the information output part outputs the disease prediction information.

### (Supplementary Note 2)

The disease prediction apparatus according to Supplementary Note 1, wherein
the disease prediction information includes risk score information indicating a likelihood of disease incidence.

### (Supplementary Note 3)

The disease prediction apparatus according to Supplementary Note 2, further including:
a recommended examination information generation part, wherein
the recommended examination information generation part generates recommended examination information related to an examination recommended for the subject of disease prediction based on the risk score for each of the plurality of body parts, and
the information output part outputs the recommended examination information.

### (Supplementary Note 4)

The disease prediction apparatus according to any one of Supplementary Notes 1 to 3, wherein
the disease prediction part refers to past disease prediction information, extracts a content that has changed in comparison with the past disease prediction information from current disease prediction information, and generates the content as disease prediction change information, and
the information output part outputs the disease prediction change information.

### (Supplementary Note 5)

The disease prediction apparatus according to Supplementary Note 1, further including:
a statistical analysis part, wherein
the statistical analysis part statistically analyzes the disease prediction information of a plurality of subjects of disease prediction, analyzes an association between a type of disease and an attribute of the subject of disease prediction, and generates attribute-related information, and
the information output part outputs the attribute-related information.

### (Supplementary Note 6)

The disease prediction apparatus according to Supplementary Note 5, wherein
the statistical analysis part further extracts an attribute with a high likelihood of disease incidence, based on the attribute-related information, to generate high-risk attribute information, and
the information output part outputs the high-risk attribute information.

### (Supplementary Note 7)

The disease prediction apparatus according to Supplementary Note 1, further including:
a statistical analysis part, wherein
the statistical analysis part statistically analyzes the disease prediction information of a plurality of subjects of disease prediction, analyzes a correlation between diseases, and generates disease correlation information, and
the information output part outputs the disease correlation information.

### (Supplementary Note 7A)

The disease prediction apparatus according to any one of Supplementary Notes 1 to 7, wherein
the disease prediction part predicts a disease that the subject of disease prediction may contract in the future in each of a plurality of body parts, based on the disease-related information and statistical information on risk of disease incidence at regular time intervals, to generate disease prediction information.

### (Supplementary Note 8)

A disease prediction method, including:
acquiring information,
predicting disease, and
outputting information, wherein

the acquiring information acquires disease-related information for predicting a disease for each subject of disease prediction,
the predicting disease predicts a disease that the subject of disease prediction may contract in the future in each of a plurality of body parts, based on the disease-related information, to generate disease prediction information, and
the outputting information outputs the disease prediction information,
which are executed by a computer.

### (Supplementary Note 9)

The disease prediction method according to Supplementary Note 8, wherein
the disease prediction information includes risk score information indicating a likelihood of disease incidence.

### (Supplementary Note 10)

The disease prediction method according to Supplementary Note 9, further including:
generating recommended examination information, wherein
the generating recommended examination information generates recommended examination information related to an examination recommended for the subject of disease prediction based on the risk score for each of the plurality of body parts, and
the outputting information outputs the recommended examination information,
which are executed by a computer.

### (Supplementary Note 11)

The disease prediction method according to any one of Supplementary Notes 8 to 10, wherein
the predicting disease refers to past disease prediction information, extracts a content that has changed in comparison with the past disease prediction information from current disease prediction information, and generates the content as disease prediction change information, and
the outputting information outputs the disease prediction change information,
which are executed by a computer.

### (Supplementary Note 12)

The disease prediction method according to Supplementary Note 8, further including:
statistical analyzing, wherein
the statistical analyzing statistically analyzes the disease prediction information of a plurality of subjects of disease prediction, analyzes an association between a type of disease and an attribute of the subject of disease prediction, and generates attribute-related information, and
the outputting information outputs the attribute-related information,
which are executed by a computer.

### (Supplementary Note 13)

The disease prediction method according to Supplementary Note 12, wherein
the statistical analyzing further extracts an attribute with a high likelihood of disease incidence, based on the attribute-related information, to generate high-risk attribute information, and
the outputting information outputs the high-risk attribute information,
which are executed by a computer.

### (Supplementary Note 14)

The disease prediction method according to Supplementary Note 8, further including:
statistical analyzing, wherein
the statistical analyzing statistically analyzes the disease prediction information of a plurality of subjects of disease prediction, analyzes a correlation between diseases, and generates disease correlation information, and
the outputting information outputs the disease correlation information,
which are executed by a computer.

### (Supplementary Note 14A)

The disease prediction method according to any one of Supplementary Notes 8 to 14, wherein
the predicting disease predicts a disease that the subject of disease prediction may contract in the future in each of a plurality of body parts, based on the disease-related information and statistical information on risk of disease incidence at regular time intervals, to generate disease prediction information.

### (Supplementary Note 15)

A program for causing a computer to execute procedures, including:
an information acquisition procedure;
a disease prediction procedure; and
an information output procedure, wherein
the information acquisition procedure acquires disease-related information for predicting a disease for each subject of disease prediction,
the disease prediction procedure predicts a disease that the subject of disease prediction may contract in the future in each of a plurality of body parts, based on the disease-related information, to generate disease prediction information, and
the information output procedure outputs the disease prediction information.

### (Supplementary Note 16)

The program according to Supplementary Note 15, wherein
the disease prediction information includes risk score information indicating a likelihood of disease incidence.

### (Supplementary Note 17)

The program according to Supplementary Note 16, further including:
a recommended examination information generation procedure, wherein
the recommended examination information generation procedure generates recommended examination information related to an examination recommended for the subject of disease prediction based on the risk score for each of the plurality of body parts, and
the information output procedure outputs the recommended examination information.

### (Supplementary Note 18)

The program according to any one of Supplementary Notes 15 to 17, wherein
the disease prediction procedure refers to past disease prediction information, extracts a content that has changed in comparison with the past disease prediction information from current disease prediction information, and generates the content as disease prediction change information, and
the information output procedure outputs the disease prediction change information,
which are executed by a computer.

### (Supplementary Note 19)

The program according to Supplementary Note 15, further including:
a statistical analysis procedure, wherein
the statistical analysis procedure statistically analyzes the disease prediction information of a plurality of subjects of disease prediction, analyzes an association between a type of disease and an attribute of the subject of disease prediction, and generates attribute-related information, and
the information output procedure outputs the attribute-related information,
which are executed by a computer.

### (Supplementary Note 20)

The program according to Supplementary Note 19, wherein
the statistical analysis procedure further extracts an attribute with a high likelihood of disease incidence, based on the attribute-related information, to generate high-risk attribute information, and
the information output procedure outputs the high-risk attribute information,
which are executed by a computer.

### (Supplementary Note 21)

The program according to Supplementary Note 15, further including:
a statistical analysis procedure, wherein
the statistical analysis procedure statistically analyzes the disease prediction information of a plurality of subjects of disease prediction, analyzes a correlation between diseases, and generates disease correlation information, and
the information output procedure outputs the disease correlation information,
which are executed by a computer.

### (Supplementary note 21A)

The program according to any one of Supplementary Notes 15 to 21, wherein
the disease prediction procedure predicts a disease that the subject of disease prediction may contract in the future in each of a plurality of body parts, based on the disease-related information and statistical information on risk of disease incidence at regular time intervals, to generate disease prediction information,
which are executed by a computer.

### (Supplementary Note 22)

A computer-readable recording medium recorded with a program for causing a computer to execute procedures, wherein the program includes:
an information acquisition procedure;
a disease prediction procedure; and
an information output procedure, wherein
the information acquisition procedure acquires disease-related information for predicting a disease for each subject of disease prediction,
the disease prediction procedure predicts a disease that the subject of disease prediction may contract in the future in each of a plurality of body parts, based on the disease-related information, to generate disease prediction information, and
the information output procedure outputs the disease prediction information.

### (Supplementary Note 23)

The computer-readable recording medium according to Supplementary Note 22, wherein
the disease prediction information includes risk score information indicating a likelihood of disease incidence.

### (Supplementary Note 24)

The computer-readable recording medium according to Supplementary Note 23, wherein the program further includes:
a recommended examination information generation procedure, wherein
the recommended examination information generation procedure generates recommended examination information related to an examination recommended for the subject of disease prediction based on the risk score for each of the plurality of body parts, and
the information output procedure outputs the recommended examination information.

### (Supplementary Note 25)

The computer-readable recording medium according to any one of Supplementary Notes 22 to 24, wherein
the disease prediction procedure refers to past disease prediction information, extracts a content that has changed in comparison with the past disease prediction information from current disease prediction information, and generates the content as disease prediction change information, and
the information output procedure outputs the disease prediction change information.

### (Supplementary Note 26)

The computer-readable recording medium according to Supplementary Note 22, wherein the program further includes:
a statistical analysis procedure, wherein
the statistical analysis procedure statistically analyzes the disease prediction information of a plurality of subjects of disease prediction, analyzes an association between a type of disease and an attribute of the subject of disease prediction, and generates attribute-related information, and
the information output procedure outputs the attribute-related information.

### (Supplementary Note 27)

The computer-readable recording medium according to Supplementary Note 26, wherein
the statistical analysis procedure further extracts an attribute with a high likelihood of disease incidence, based on the attribute-related information, to generate high-risk attribute information, and
the information output procedure outputs the high-risk attribute information.

### (Supplementary Note 28)

The computer-readable recording medium according to Supplementary Note 22, wherein the program further includes:
a statistical analysis procedure, wherein
the statistical analysis procedure statistically analyzes the disease prediction information of a plurality of subjects of disease prediction, analyzes a correlation between diseases, and generates disease correlation information, and
the information output procedure outputs the disease correlation information.

### (Supplementary Note 28A)

The computer-readable recording medium according to any one of Supplementary Notes 22 to 28, wherein
the disease prediction procedure predicts a disease that the subject of disease prediction may contract in the future in each of a plurality of body parts, based on the disease-related information and statistical information on risk of disease incidence at regular time intervals, to generate disease prediction information.

This application claims priority from Japanese Patent Application No. 2023-068450 filed on April 19, 2023, Japanese Patent Application No. 2023-180785 filed on October 20, 2023, and Japanese Patent Application No. 2024-022230 filed on February 16, 2024. The entire subject matters of the Japanese Patent Applications are incorporated herein by reference.

### Industrial Applicability

According to the present disclosure, for example, it is possible to obtain, from information on diseases such as health checkup, disease prediction information for each of a plurality of body parts of the subject of disease prediction, recommended examination information, and information related to statistical analysis results. Thus, the subjects of disease prediction can comprehensively grasp their health condition, and the present disclosure is useful for appropriate health management of the subjects of disease prediction.

### Reference Signs List

- 10A, 10B, 10C:: disease prediction apparatus
- 11:: information acquisition part
- 12:: disease prediction part
- 13:: information output part
- 14:: recommended examination information generation part
- 15:: statistical analysis part
- 101:: central processing unit
- 102:: memory
- 103:: bus
- 104:: storage
- 105:: input unit
- 106:: output unit
- 107:: communication device

## Claims

1. A disease prediction apparatus, comprising:
an information acquisition part;
a disease prediction part; and
an information output part, wherein
the information acquisition part acquires disease-related information for predicting a disease for each subject of disease prediction,
the disease prediction part predicts a disease that the subject of disease prediction may contract in the future in each of a plurality of body parts, based on the disease-related information, to generate disease prediction information, and
the information output part outputs the disease prediction information.

2. The disease prediction apparatus according to claim 1, wherein
the disease prediction information includes risk score information indicating a likelihood of disease incidence.

3. The disease prediction apparatus according to claim 2, further comprising:
a recommended examination information generation part, wherein
the recommended examination information generation part generates recommended examination information related to an examination recommended for the subject of disease prediction based on the risk score for each of the plurality of body parts, and
the information output part outputs the recommended examination information.

4. The disease prediction apparatus according to any one of claims 1 to 3, wherein
the disease prediction part refers to past disease prediction information, extracts a content that has changed in comparison with the past disease prediction information from current disease prediction information, and generates the content as disease prediction change information, and
the information output part outputs the disease prediction change information.

5. The disease prediction apparatus according to claim 1, further comprising:
a statistical analysis part, wherein
the statistical analysis part statistically analyzes the disease prediction information of a plurality of subjects of disease prediction, analyzes an association between a type of disease and an attribute of the subject of disease prediction, and generates attribute-related information, and
the information output part outputs the attribute-related information.

6. The disease prediction apparatus according to claim 5, wherein
the statistical analysis part further extracts an attribute with a high likelihood of disease incidence, based on the attribute-related information, to generate high-risk attribute information, and
the information output part outputs the high-risk attribute information.

7. The disease prediction apparatus according to claim 1, further comprising:
a statistical analysis part, wherein
the statistical analysis part statistically analyzes the disease prediction information of a plurality of subjects of disease prediction, analyzes a correlation between diseases, and generates disease correlation information, and
the information output part outputs the disease correlation information.

8. The disease prediction apparatus according to any one of claims 1 to 7, wherein
the disease prediction part predicts a disease that the subject of disease prediction may contract in the future in each of a plurality of body parts, based on the disease-related information and statistical information on risk of disease incidence at regular time intervals, to generate disease prediction information.

9. A disease prediction method, comprising:
acquiring information,
predicting disease, and
outputting information, wherein
the acquiring information acquires disease-related information for predicting a disease for each subject of disease prediction,
the predicting disease predicts a disease that the subject of disease prediction may contract in the future in each of a plurality of body parts, based on the disease-related information, to generate disease prediction information, and
the outputting information outputs the disease prediction information,
which are executed by a computer.

10. The disease prediction method according to claim 9, wherein
the disease prediction information includes risk score information indicating a likelihood of disease incidence.

11. The disease prediction method according to claim 10, further comprising:
generating recommended examination information, wherein
the generating recommended examination information generates recommended examination information related to an examination recommended for the subject of disease prediction based on the risk score for each of the plurality of body parts, and
the outputting information outputs the recommended examination information,
which are executed by a computer.

12. The disease prediction method according to any one of claims 9 to 11, wherein
the predicting disease refers to past disease prediction information, extracts a content that has changed in comparison with the past disease prediction information from current disease prediction information, and generates the content as disease prediction change information, and
the outputting information outputs the disease prediction change information,
which are executed by a computer.

13. The disease prediction method according to claim 9, further comprising:
statistical analyzing, wherein
the statistical analyzing statistically analyzes the disease prediction information of a plurality of subjects of disease prediction, analyzes an association between a type of disease and an attribute of the subject of disease prediction, and generates attribute-related information, and
the outputting information outputs the attribute-related information,
which are executed by a computer.

14. The disease prediction method according to claim 13, wherein
the statistical analyzing further extracts an attribute with a high likelihood of disease incidence, based on the attribute-related information, to generate high-risk attribute information, and
the outputting information outputs the high-risk attribute information,
which are executed by a computer.

15. The disease prediction method according to claim 9, further comprising:
statistical analyzing, wherein
the statistical analyzing statistically analyzes the disease prediction information of a plurality of subjects of disease prediction, analyzes a correlation between diseases, and generates disease correlation information, and
the outputting information outputs the disease correlation information,
which are executed by a computer.

16. The disease prediction method according to any one of claims 9 to 16, wherein
the predicting disease predicts a disease that the subject of disease prediction may contract in the future in each of a plurality of body parts, based on the disease-related information and statistical information on risk of disease incidence at regular time intervals, to generate disease prediction information.

17. A program for causing a computer to execute procedures, comprising:
an information acquisition procedure;
a disease prediction procedure; and
an information output procedure, wherein
the information acquisition procedure acquires disease-related information for predicting a disease for each subject of disease prediction,
the disease prediction procedure predicts a disease that the subject of disease prediction may contract in the future in each of a plurality of body parts, based on the disease-related information, to generate disease prediction information, and
the information output procedure outputs the disease prediction information.

18. The program according to claim 17, wherein
the disease prediction information includes risk score information indicating a likelihood of disease incidence.

19. The program according to claim 18, further comprising:
a recommended examination information generation procedure, wherein
the recommended examination information generation procedure generates recommended examination information related to an examination recommended for the subject of disease prediction based on the risk score for each of the plurality of body parts, and
the information output procedure outputs the recommended examination information.

20. A computer-readable recording medium recorded with a program for causing a computer to execute procedures, wherein the program comprises:
an information acquisition procedure;
a disease prediction procedure; and
an information output procedure, wherein
the information acquisition procedure acquires disease-related information for predicting a disease for each subject of disease prediction,
the disease prediction procedure predicts a disease that the subject of disease prediction may contract in the future in each of a plurality of body parts, based on the disease-related information, to generate disease prediction information, and
the information output procedure outputs the disease prediction information.
